# EUROPEAN PATENT SPECIFICATION

(11) **EP 1 280 506 B1**
(45) Date of publication and mention of the grant of the patent: **21.06.2006**
(21) Application number: 01945078.2
(22) Date of filing: 03.05.2001
(51) Int. Cl.: A61K 8/58, A61Q 19/04

(54) **SILYLATED COMPOUNDS AS PRECURSORS FOR SELF-TANNING COMPOSITIONS**
SILYLIERTE VERBINDUNGEN ALS VORLÄUFER FÜR SELBSTBRÄUNUNGS ZUSAMMENSETZNGEN
COMPOSES SILYLATES UTILISES COMME PRECURSEURS DANS DES COMPOSITIONS AUTO-BRONZANTES

(30) Priority: 10.05.2000 EP 00810400
(43) Date of publication of application: 05.02.2003
(73) Proprietor: Ciba Specialty Chemicals Holding Inc., 4057 Basel (CH)
(72) Inventor: EHLIS, Thomas, 79106 Freiburg (DE); HÜGLIN, Dietmar, 79591 Eimeldingen (DE); RÖDING, Joachim, Friedrich, 22765 Hamburg (DE)
(86) International application number: PCT/EP2001/004983
(87) International publication number: WO 2001/085124

(56) References cited:
- WO-A-97/33560
- US-A- 4 956 174
- JEONG, L. S. ET AL.: NUCLEOSIDES & NUCLEOTIDES, vol. 17, no. 8, 1998, pages 1473-1487, XP000952971
- ARREGUIN LOZANO, B. ET AL.: "Gas chromatography of trimethylsilyl sugars at various temperatures" JOURNAL OF CHROMATOGRAPHIC SCIENCE, 8 April 1970 (1970-04-08), XP000926035
- CHATANI, N. ET AL.: "Reductive Oligomerisation of Carbon Monoxide by Rhodium-Catalyzed Reaction with Hydrosilanes" JOURNAL OF THE AMERICAN CHEMICAL SOCIETY, vol. 119, no. 18, 1997, XP000953176
- SERIANNI, A. S. ET AL.: "Cyanohydrin synthesis: studies with carbon-13-labeled cyanide" JOURNAL OF ORGANIC CHEMISTRY, vol. 45, no. 16, 1980, XP000673337

## Description

The present invention relates to the use of silylated compounds as precursors for self-tanning compositions, and to cosmetic formulations comprising these silylated compounds.

Skin self-tanning substances are substances which produce brown dyes upon contact with the skin as a result of a chemical reaction with the keratin in the skin without the action of natural or artificial light (J. Eichler, Kontakte (Merck, Darmstadt), 1981, 3, 24). Typically, these compounds contain aldol, ketol or dicarbonyl groups in the molecule, dihydroxyacetone (DHA) being the best known representative. The following compounds are also of importance:
glyceraldehyde, 6-aldo-D-fructose, hydroxymethylglyoxal, mucondialdehyde, malealdehyde, substituted succindialdehydes, erythrulose, dihydroxyacetone (as monomer or dimer), methylglyoxal (pyruvaldehyde), substituted 4,4'-dihydroxypyrazolin-5-ones, as described in EP-A-0903342.

These compounds react with free amino groups in the skin in a Maillard reaction to give brown-coloured substances in the stratum corneum. This reaction is complete after 4 to 12 hours. The tan achieved cannot be washed off and is removed only with normal skin desquamation, i.e. it takes approximately 5 to 15 days until the skin is completely decoloured.

However, the practical use of self-tanning active ingredients, for example DHA, in self-tanning compositions has disadvantages, such as inadequate stability and high reactivity with other formulation components (discoloration, formation of unpleasant odours), poor solubility in nonpolar organic solvents (virtually insoluble) and difficult formulatability.

Document JEONG, L. S. ET AL.: NUCLEOSIDES & NUCLEOTIDES, vol. 17, no. 8, 1998, pages 1473-1487, XP000952971, discloses the synthesis of the t-butyldimethylsilyl derivative of dihydroxyacetone. No skin tanning properties are disclosed.

Surprisingly, it has been found that by silylating the skin self-tanning substances listed above, it is possible to prepare precursors of these substances which no longer have these disadvantages. Following hydrolysis or enzymatic cleavage of these active ingredient precursors on the skin, the self-tanning substances are again released.

The present invention thus provides for the use of silylated derivatives as precursors of skin self-tanning substances in self-tanning compositions.

In particular, the partially or completely silylated derivatives of the following skin self-tanning substances are of importance:
glyceraldehyde, 6-aldo-D-fructose, hydroxymethylglyoxal, mucondialdehyde, malealdehyde, substituted succindialdehydes, erythrulose, dihydroxyacetone (as monomer or dimer), methylglyoxal (pyruvaldehyde), substituted 4,4'-dihydroxypyrazolin-5-ones, as described in EP-A-0903342.

Preference is given according to the invention to using compounds of the formulae 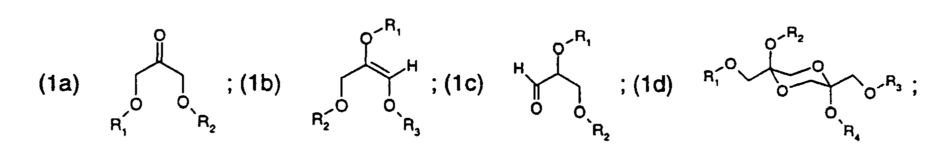 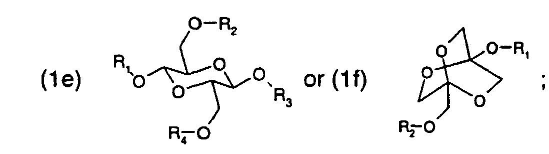 in which
R₁, R₂, R₃ and R₄ independently of one another are hydrogen or a radical of the formula 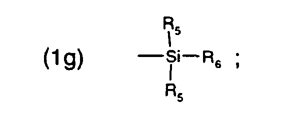
R₅ is C₁-C₅alkyl; aryl; C₆-C₁₀aryl; and
R₆ C₁-C₁₆alkyl;
   where
   in the formulae (1 a) to (1f) at least one of the radicals R₁, R₂, R₃ and R₄ is a radical of the formula (1g).

C₁-C₅Alkyl and C₁-C₁₆alkyl are straight-chain or branched alkyl radicals, for example methyl, ethyl, n-propyl, isopropyl, n-butyl, sec-butyl, tert-butyl, amyl, isoamyl or tert-amyl, heptyl, octyl, isooctyl, nonyl, decyl, undecyl, dodecyl, tetradecyl, pentadecyl or hexadecyl.

C₆-C₁₀Aryl is naphthyl and preferably phenyl.

Particular preference is given according to the invention to using compounds of the formulae 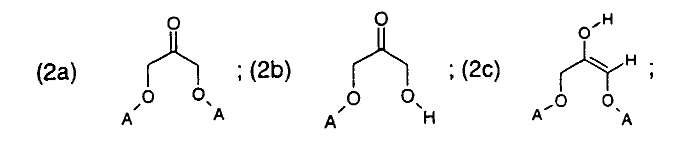 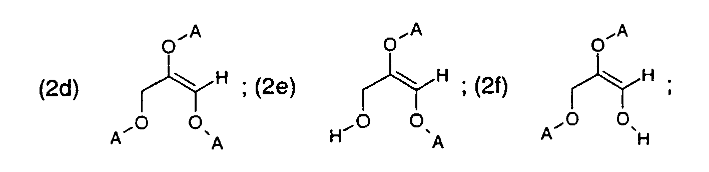 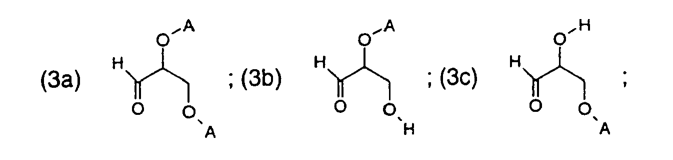 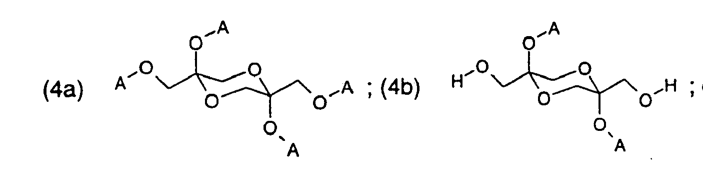 or 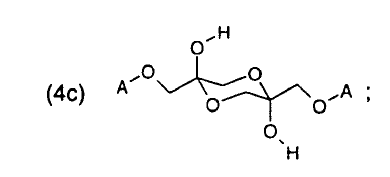 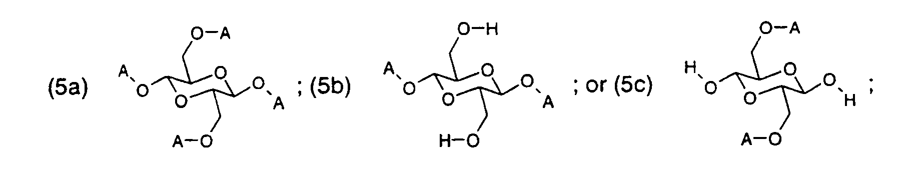 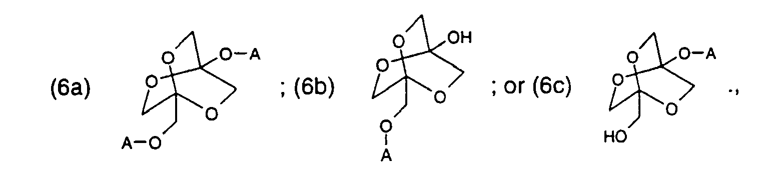 in which, in the formulae given above,
A is a radical of the formula (1 g).

Particular preference is given to using compounds in which, in formula (1g),
R₅ is methyl or ethyl.

Very particular preference is given according to the invention to using the compounds of the formulae

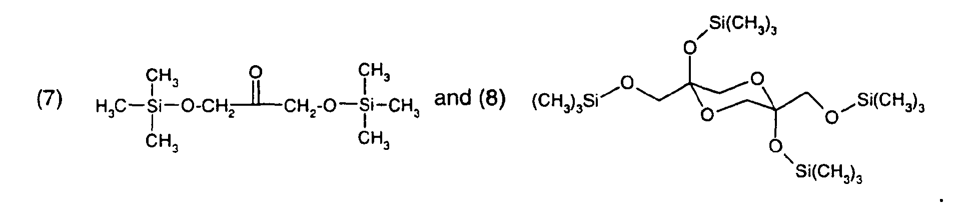

The soluble precursors of skin self-tanning substances used according to the invention are prepared in a manner known per se. The process comprises silylating the skin self-tanning substances glyceraldehyde, 6-aldo-D-fructose, hydroxymethylglyoxal, mucondialdehyde, malealdehyde, substituted succindialdehydes, erythrulose, dihydroxyacetone, methylglyoxal (pyruvaldehyde), substituted 4,4'-dihydroxypyrazolin-5-ones with a suitable silylating agent.

The process for the preparation of the compounds of the formulae (1 a) to (1f) comprises reacting dihydroxyacetone in monomeric or dimeric form with a suitable silylating reagent.

Some of the compounds of the formulae (1) to (8) are those of the formulae 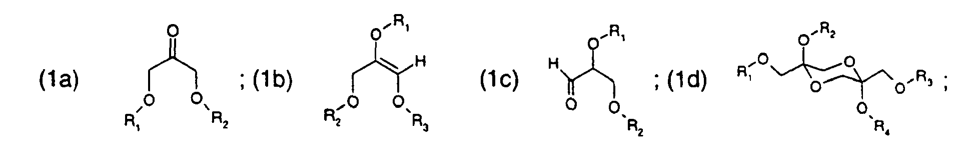 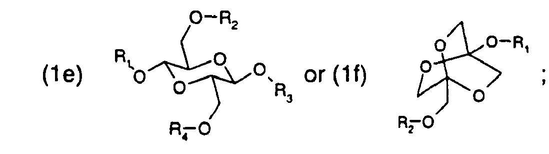 in which
R₁, R₂, R₃ and R₄ independently of one another are hydrogen or a radical of the formula 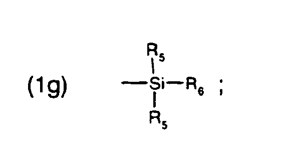
R₅ is C₁-C₅alkyl; aryl; C₆-C₁₀aryl; and
R₆ C₁-C₁₆alkyl;
where
in the formulae (1a) to (1f) at least one of the radicals R₁, R₂, R₃ and R₄ is a radical of the formula (1 g), and the following compounds are not included:
- compounds of the formula (1a), in which A and R₁ are at the same time a radical of the formula 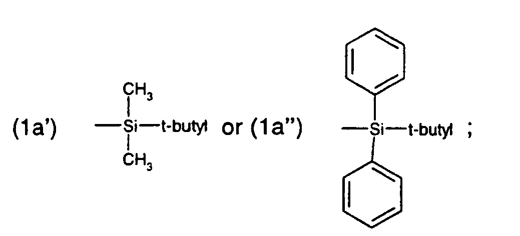
- compounds of the formula (1 a) in which A is a radical of the formulae (1 a') or (1 a") and R₁ is hydrogen;
- compounds of the formula (1 c) in which R₁ and R₂ are a radical of the formula (1a');
- compounds of the formula (1 d) in which R₁, R₂, R₃ and R₄ are a radical of the formula

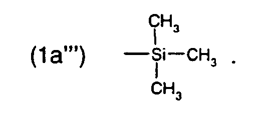

The compounds are prepared by reacting dihydroxyacetone, which is in crystalline form as a dimer of the formula 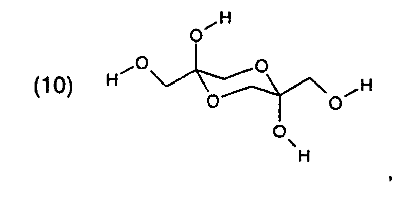 with a silylating reagent of the formula 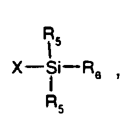 in which
X is halogen, -CN, -NHSi(R₅)₂R₆, -OSi(R₅)₂R₆, -N(CH₂CH₃)₂, -N(CH₃)₂, -NH(CO)NHSi(R₅)₂R₆
   and
R₅ and R₆ are as defined above.

Through suitable choice of the reaction conditions during the silylation reaction it is possible to obtain the compounds of the formulae (1a) to (1f).

The reaction temperature is between -40 and 150°C, preferably 0 and 100°C.

Suitable solvents are halogenated aliphatic and aromatic hydrocarbons, for example chlorobenzene, dichlorobenzene, dichloromethane, trichloromethane or tetrachloromethane, dipolar aprotic solvents, for example dimethylformamide, sulfolane or acetonitrile, ethers, for example tetrahydrofuran, diethyl ether, tert-butyl methyl ether or dioxane, aromatic hydrocarbons, for example toluene, xylene or benzene, or aliphatic hydrocarbons, for example petroleum ether or hexane.

The reactions can also be carried out without solvents by using an excess of the silylating reagent.

Auxiliary bases used are inorganic and/or organic bases, for example triethylamine, trimethylamine, ethyldiisopropylamine, pyridine or imidazole.

Silylating reagents 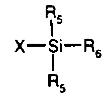 and methods are generally known and described in

E.P. Pluddemann, Kirk-Othmer, Encycl. Chem. Technol., 3^{rd} ed., Vol. 20, John Wiley & Sons, New York, 1982; T. W. Greene, P. G. M. Wuts, Protective Groups in Organic Synthesis, 3^{rd} ed., John Wiley & Sons, New York, 1999 and G. van Look, G. Simchen, J. Heberle, Silylating Agents, 2^{nd} ed., Fluka Chemie AG, Buchs, 1995.

The invention further provides a cosmetic formulation comprising 2 to 15% by weight, preferably 5 to 10% by weight, of a precursor or of two or more precursors of the formula (1), and customary cosmetic carriers or auxiliaries.

Because of its physicochemical properties, the precursor is sensitive to hydrolysis and cannot therefore be used in the customarily used cosmetic W/O or O/W formulations or in other customary cosmetic formulations in which the water phase comes into direct contact with the phase which contains the precursor.

Of particular suitability, therefore, for incorporating the precursor are water-free formulations or systems in which the water-containing phase and the active ingredient phase are not in direct contact with one another and are only mixed together upon application (2-chamber or multichamber systems).

Water-free systems which can be used are, for example, oils based on triglycerides or mineral oils, and also silicone oils in the form of sprays or as liquid. Silicone oils may also be used in semisolid form, in the form of "silicone gels". A further variant is oleogels which contain thickened triglycerides. Likewise suitable are water-free powders and aerosols.

The customary cosmetic carriers or auxiliaries which are present in the cosmetic composition according to the invention include various oil components, such as fats, oils, silicone oil etc., further substances such as allantoin, thickeners, water-free preservatives, perfume oils, antioxidants, for example vitamin E, carotinoids or HALS compounds.

Moreover, the composition according to the invention can also comprise further UV protection substances from the following classes of substance:
1. p-aminobenzoic acid derivatives, for example 2-ethylhexyl 4-dimethylaminobenzoate;
2. salicylic acid derivatives, for example 2-ethylhexyl salicylate;
3. benzophenone derivatives, for example 2-hydroxy-4-methoxybenzophenone and its 5-sulfonic acid derivative;
4. dibenzoylmethane derivatives, for example 1-(4-tert-butylphenyl)-3-(4-methoxyphenyl)propane-1,3-dione;
5. diphenyl acrylates, for example 2-ethylhexyl 2-cyano-3,3-diphenyl acrylate and 3-benzofuranyl 2-cyanoacrylate;
6. 3-imidazol-4-ylacrylic acid and esters;
7. benzofuran derivatives, in particular 2-(p-aminophenyl)benzofuran derivatives, described in EP-A-582,189, US-A-5,338,539, US-A-5,518,713 and EP-A-613,893;
8. polymeric UV absorbers, for example the benzylidene malonate derivatives described in EP-A-709,080;
9. cinnamic acid derivatives, for example the 2-ethylhexyl 4-methoxycinnamate or isoamyl 4-methoxycinnamate disclosed in US-A-5,601,811 and WO 97/00851;
10. camphor derivatives, for example 3-(4'-methyl)benzylidenebornan-2-one, 3-benzylidenebornan-2-one, N-[2(and 4)-2-oxyborn-3-ylidenemethyl)benzyl]acrylamide polymer, 3-(4'-trimethylammonium)benzylidenebornan-2-one methylsulfate, 3,3'-(1,4-phenylenedimethine)bis(7,7-dimethyl-2-oxobicyclo[2.2.1]heptan-1-methanesulfonic acid) and salts, 3-(4'-sulfo)benzylidenebornan-2-one and salts;
11. trianilino-s-triazine derivatives, for example 2,4,6-trianilino(p-carbo-2'-ethyl-1'-oxy)-1,3,5-triazine, and the UV absorbers disclosed in US-A-5,332,568, EP-A-517,104, EP-A-507,691, WO 93/17002 and EP-A-570,838;
12. 2-hydroxyphenylbenzotriazole derivatives;
13. hydroxyphenyl-s-triazine compounds, for example the compounds described in US-A-5,955,060;
14. 2-phenylbenzimidazole-5-sulfonic acid and salts thereof;
15. menthyl o-aminobenzoate;
16. TiO₂ (with various coatings), ZnO and mica.

The UV absorbers described in "Sunscreens", Eds. N.J. Lowe, N.A.Shaath, Marcel Dekker, Inc. , New York and Basle or in Cosmetics & Toiletries (107), 50 ff (1992) can also be used as additional UV protection substances in the cosmetic composition according to the invention.

To improve the shade of brown, the substances/formulations according to the invention can also be used together with amino acids (e.g. tryptophan, cysteine, histidine, arginine, lysine, tyrosine, aspartic acid, methionine, proline, phenylalanine, hydroxyproline, leucine, glycine, isoleucine, threonine, serine, valine, alanine, cystine).

The examples below illustrate the invention.

### General procedure

1 equivalent of the skin self-tanning compound is suspended in dichloromethane. 1.1 equivalents of triethylamine are added per hydroxyl group, and 1.1 equivalents of trimethylchlorosilane per hydroxyl group are added dropwise at room temperature. The mixture is then stirred for 16h at RT, treated with some kieselguhr and filtered. The filtrate is evaporated under reduced pressure at 30°C. The residue is treated with hexane and thoroughly stirred for 30 min under a protective gas. Insoluble constituents are filtered off and the filtrate is evaporated at 30°C under reduced pressure.
This gives solids, oils or liquids which release the skin self-tanning active ingredients after contact with water.

### Example 1: Preparation of dihydroxyacetone derivatives

Dihydroxyacetone (45.1 g, 0.5 mol) in the form of the dimer and triethylamine (106.3 g, 1.05 mol) are suspended in dichloromethane (400 ml) and, at about 5°C, a solution of trimethylchlorosilane (108.6 g, 1.0 mol) in 100 ml of dichloromethane are slowly added dropwise. The mixture is then stirred for 16 h at room temperature, treated with some kieselguhr and filtered. The filtrate is concentrated by evaporation under reduced pressure at 30°C. The residue is treated with 200 ml of hexane and thoroughly stirred for 30 min under a protective gas. Insoluble constituents are filtered off and the filtrate is concentrated by evaporation at 30°C at reduced pressure.
The colourless liquid which remains (mixture of various silylation products of dihydroxyacetone) releases skin self-tanning active ingredients after contact with water.

Bulb-tube distillation in a high vacuum or fractional distillation in a high vacuum are used to isolate individual compounds.

Furthermore, separation into the individual components can be carried out by preparative column chromatography.
1,3-bis(Trimethylsiloxy)-2-propanone is isolated as colourless liquid.
Yield: 38%
b.p. (0.46-0.27 mbar): 44-51 °C
¹H-NMR (360 MHz, CDCl₃): δ= 0.01 (s, 18H, CH₃), 4.24 (s, 4H, CH₂)
¹³C-NMR (90 MHz, CDCl₃): δ= 0.16 (CH₃), 67.43 (CH₂), 209 (C=O).

### Example 2: Demonstration of the action

The colour development upon contact with water is demonstrated in the in vitro test described by M. F. Bobin et al. (M. F. Bobin, M. C. Martini, J. Cotte, J. Soc. Cosmet. Chem., 1984, 35, 265).

0.02 mol/l of lysine are dissolved in a phosphate buffer system in accordance with DIN 19268 (pH 7). 10 ml of this solution are treated with 0.002 mol of 1,3-bis(trimethylsiloxy)-2-propanone and the mixture is stirred at 32-35°C for 72 h.

As comparison, 10 ml of the lysine solution are treated with 0.002 mol of dihydroxyacetone and stirred at 32 - 35°C for 72 h.

In both cases a comparable brown coloration is obtained after 72 h. In the case of the use of 1,3-bis(trimethylsiloxy)-2-propanone, the brown coloration develops more slowly.

Comparable brown colorations are also obtained if, instead of 1,3-bis(trimethylsiloxy)-2-propanone, the crude product from the reaction between dihydroxyacetone and trimethylchlorosilane liquid (mixture of various silylation products of dihydroxyacetone) is used.

### Example 3: Water-free formulations

Instead of 1,3-bis(trimethylsiloxy)-2-propanone it is also possible to use the crude product from the reaction between dihydroxyacetone and trimethylchlorosilane (mixture of different silylation products of dihydroxyacetone).
A.

| Trade name | INCI/EU | % |
|---|---|---|
| Vaseline | Petrolatum | 50-80 |
| Lanolin | Lanolin | 10-30 |
| Mineral oil | Paraffinum Liquidum | 10-30 |
| 1,3-bis(Trimethylsiloxy)-2-propane | | 5-10 |
| Tocopherol | Tocopherol | 0.1-1.0 |
| Corn germ oil | Zea Mays | 1-5 |
| Castor oil | Castor Oil | 1-5 |

B.

| Trade name/Supplier | INCI/EU | % |
|---|---|---|
| Permethyl 99A/Highpoint Chem. USA | Isododecane | 53 |
| 1,3-bis(Trimethylsiloxy)-2-propanone | | 5 |
| Permethyl 106A/Highpoint Chem. USA | Polyisobutene | 2 |
| Gel Base/Brooks | Isododecane&Mixed Ethylene Copolymer | ad 100 |

C.

| Trade name | INCI/EU | % |
|---|---|---|
| Calendula oil | Calendula officinalis | 3.00 |
| Mineral oil | Paraffinum Liquidum | 30.90 |
| 1,3-bis(Trimethylsiloxy)-2-propanone | | 5.0 |
| Paprika extract, oil-soluble | Capsicum annuum | 0.10 |
| Cetiol HE | PEG-7 Glyceryl Cocoate | 29.00 |
| Glycerol | Glycerin | 29.00 |
| Algen liquid NV | Propylene Glycol & Algae extract | 2.80 |
| Perfume | | 0.20 |

D.

| Trade name | INCI/EU | % |
|---|---|---|
| Soya oil | Glycine Soja | 60-80 |
| Myritol 318 | Caprylic/Capric Triglyceride | 15-30 |
| Sunflower oil | Helianthus annuus | 10-20 |
| 1,3-bis(Trimethylsiloxy)-2-propanone | | 5-15 |
| Com germ oil | Zea Mays | 1-5 |

E.

| Trade name | INCI/EU | % |
|---|---|---|
| IPP | Isopropyl Palmitate | 50-70 |
| DC 345 | Cyclomethicone | 20-40 |
| 1,3-bis(Trimethylsiloxy)-2-propanone | | 5.0 |
| Rapeseed oil | Brassica Oleifera | 1-5 |
| Tocopherol | Tocopherol | 0.1-1.0 |
| Saffran oil | Carthamus Tinctorius | 0.1-1.0 |
| Bisabolol | Bisabolol | 0.1-0.5 |
| Soya oil | Glycine Soja | 0.1-0.5 |

F.

| Trade name | INCI | % |
|---|---|---|
| Cetiol HE | PEG-7 Glyceryl Cocoate | 70.00 |
| Myritol 318 | Caprylic/Capric Triglyceride | 21.50 |
| 1,3-bis(Trimethylsiloxy)-2-propanone | | 5.0 |
| Aerosil 200 | Silica | 3.00 |
| Perfume | | 0.50 |

G.

| Trade name | INCI/EU | % |
|---|---|---|
| DC 345 | Cyclomethicone | 80-90 |
| DC 9506 Powder | Dimethicone/Vinyl Dimethicone Crosspolymer | 10-20 |
| 1,3-bis(Trimethylsiloxy)-2-propanone | | 2-15 |
| Perfume | | 0.5-2.0 |

H.

| Trade name | INCI/EU | % |
|---|---|---|
| Talc | Talc | 94 |
| Magnesium carbonate | Magnesium Carbonate | 1 |
| 1,3-bis(Trimethylsiloxy)-2-propanone | | 5 |

## Claims

1. The use of silylated derivatives of glyceraldehyde, 6-aldo-D-fructose,
hydroxymethylglyoxal, mucondialdehyde, malealdehyde, substituted succindialdehydes, erythrulose, dihydroxyacetone, methylglyoxal (pyruvaldehyde) and substituted 4,4'-dihydroxypyrazolin-5-ones as precursors of skin self-tanning substances in self-tanning compositions.

2. The use according to claim 1, which relates to compounds of the formulae 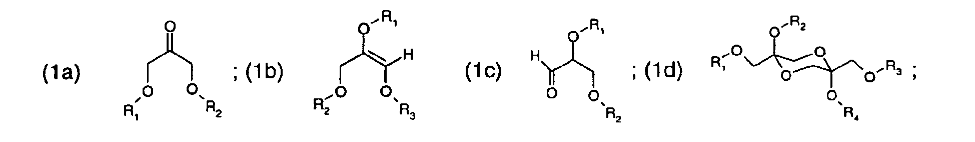 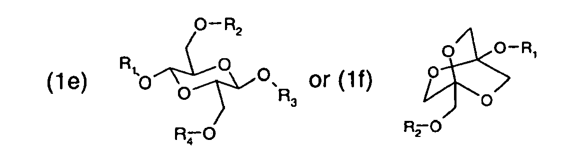 in which
R₁, R₂, R₃ and R₄ independently of one another are hydrogen or a radical of the formula 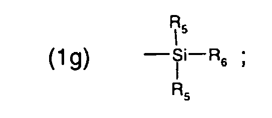
R₅ is C₁-C₅alkyl; aryl; C₁-C₃aryl; and
R₆ C₁-C₁₆alkyl;
where
in the formulae (1a) to (1f) at least one of the radicals R₁, R₂, R₃ and R₄ is a radical of the formula (1g).

3. The use according to claim 2, which relates to compounds of the formulae 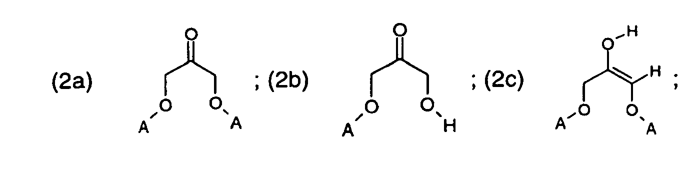 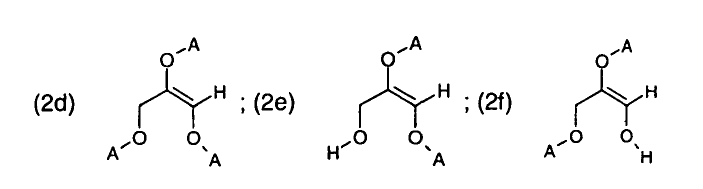 in which
A is a radical of the formula (1 g).

4. The use according to claim 2, which relates to compounds of the formulae 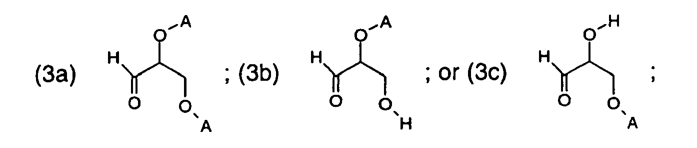 in which
A is a radical of the formula (1 g).

5. The use according to claim 2, which relates to compounds of the formulae 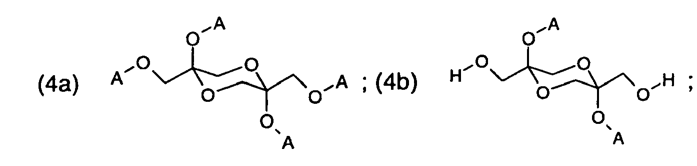 or 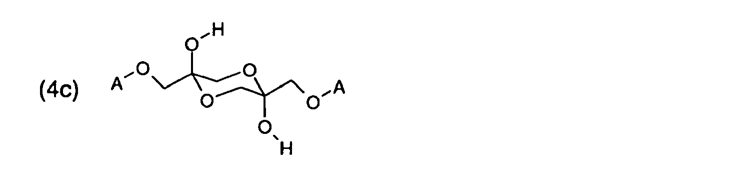 in which
A is a radical of the formula (1g).

6. The use according to claim 2, which relates to compounds of the formulae 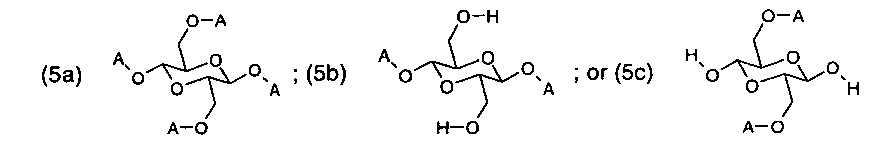 in which
A is a radical of the formula (1g).

7. The use according to claim 2, which relates to compounds of the formulae 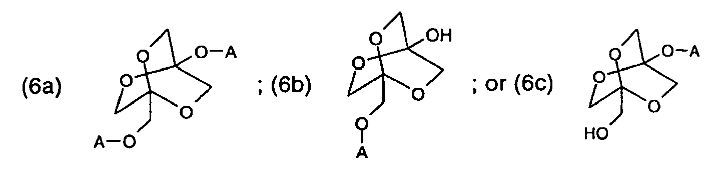 in which
A is a radical of the formula (1 g).

8. The use according to any one of claims 2 to 7, wherein, in formula (1 g),
R₅ is methyl or ethyl.

9. The use according to claim 1 or 2, which relates to the compound of the formula

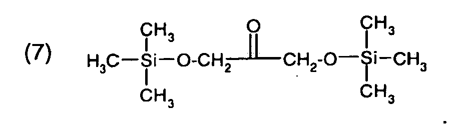

10. The use according to claim 1 or 2, which relates to the compound of the formula

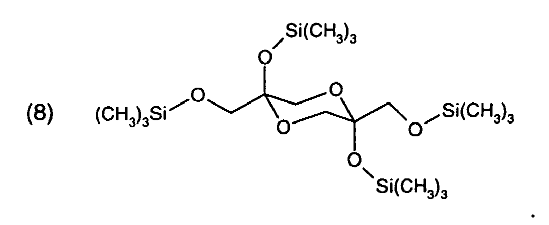

11. A cosmetic formulation comprising
2 to 15% by weight of a precursor according to claim 1, and customary cosmetic carriers or auxiliaries.

12. A cosmetic formulation according to claim 11, which is free from water.

13. A cosmetic formulation according to claim 11 or 12, which additionally comprises a UV filter.

## Patentansprüche

1. Verwendung von sylilierten Derivaten von Glycerinaldehyd, 6-Aldo-D-fructose,
Hydroxymethylglyoxal, Mucondialdehyd, Maleinaldehyd, substituierten Succindialdehyden, Erythrulose, Dihydroxyaceton, Methylglyoxal (Pyruvaldehyde) und substituierten 4,4'-Di-hydroxypyrazolin-5-onen als Vorläufer hautselbstbräunender Substanzen in Selbstbräunungsmitteln.

2. Verwendung nach Anspruch 1, betreffend Verbindungen der Formeln 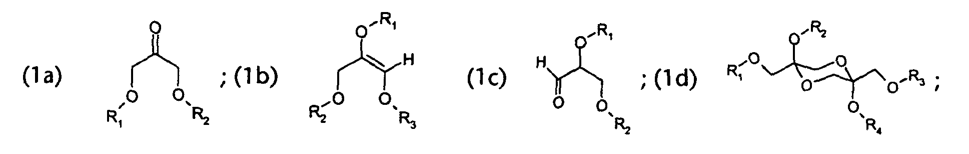 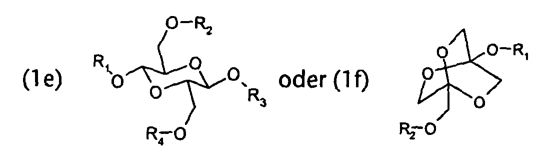 worin
R₁, R₂, R₃ und R₄ unabhängig voneinander Wasserstoff oder einen Rest der Formel 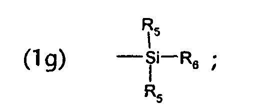
R₅ C₁-C₅-Alkyl; Aryl; C₁-C₃-Aryl; und
R₆ C₁-C₁₆-Alkyl;
bedeuten, wobei
in den Formeln (1 a) bis (1f) mindestens einer der Reste R₁, R₂, R₃ bzw. R₄ einen Rest der Formel (1 g) bedeutet.

3. Verwendung nach Anspruch 2, betreffend Verbindungen der Formeln 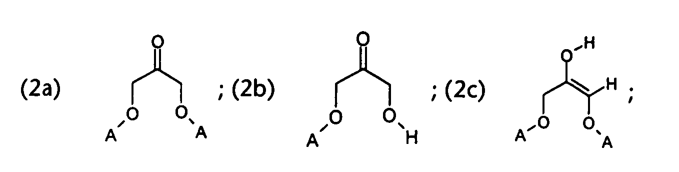 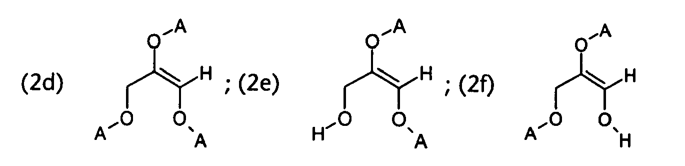 worin
A einen Rest der Formel (1 g) bedeutet.

4. Verwendung nach Anspruch 2, betreffend Verbindungen der Formeln 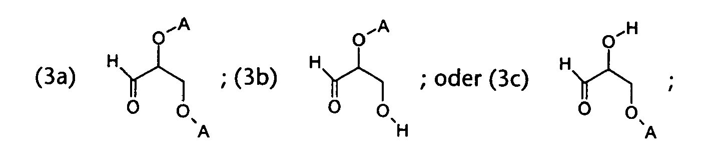 worin
A einen Rest der Formel (1 g) bedeutet.

5. Verwendung nach Anspruch 2, betreffend Verbindungen der Formeln 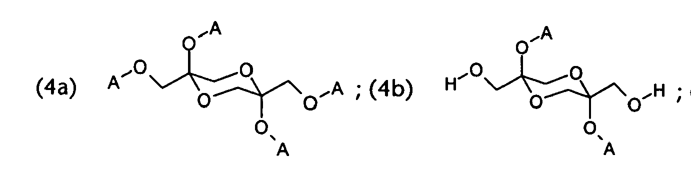 oder 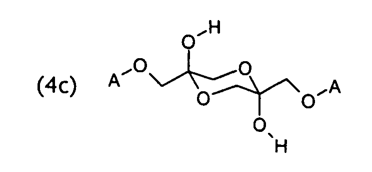 worin
A einen Rest der Formel (1g) bedeutet.

6. Verwendung nach Anspruch 2, betreffend Verbindungen der Formeln 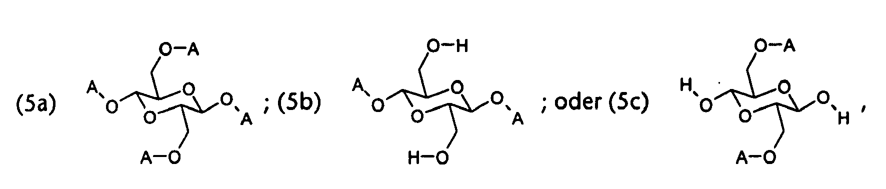 worin
A einen Rest der Formel (1 g) bedeutet.

7. Verwendung nach Anspruch 2, betreffend Verbindungen der Formeln 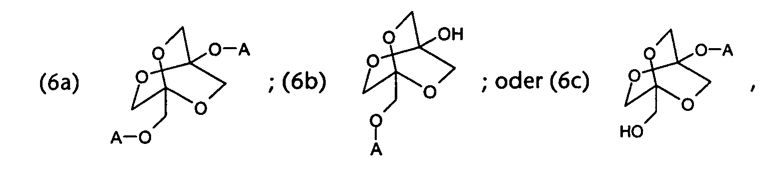 worin
A einen Rest der Formel (1 g) bedeutet.

8. Verwendung nach einem der Ansprüche 2 bis 7, worin in Formel (1 g)
R₅ Methyl oder Ethyl bedeutet.

9. Verwendung nach Anspruch 1 oder 2, betreffend die Verbindung der Formel

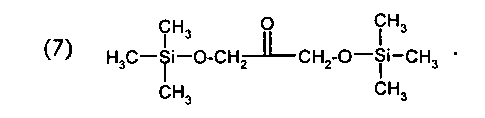

10. Verwendung nach Anspruch 1 oder 2, betreffend die Verbindung der Formel

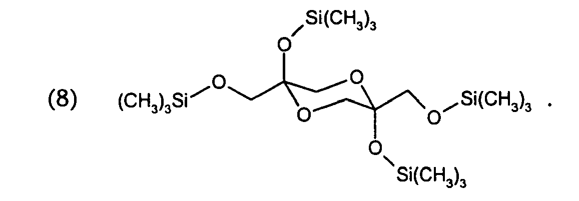

11. Kosmetische Formulierung, enthaltend 2 bis 15 Gew.-% eines Vorläufers gemäß Anspruch 1, sowie übliche kosmetische Träger- oder Hilfsstoffe.

12. Kosmetische Formulierung nach Anspruch 11, **dadurch gekennzeichnet, dass** sie wasserfrei ist.

13. Kosmetische Formulierung nach Anspruch 11 oder 12, **dadurch gekennzeichnet, dass** sie zusätzlich einen UV-Filter enthält.

## Revendications

1. Utilisation de dérivés silylés de glycéraldéhyde, de 6-aldo-D-fructose, d'hydroxyméthylglyoxal, de mucondialdéhyde, de maléaldéhyde, de succindialdéhydes substitués, d'érythrulose, de dihydroxyacétone, de méthylglyoxal (pyruvaldéhyde) et de 4,4'-dihydroxypyrazolin-5-ones substituées en tant que précurseurs de substances auto-bronzantes pour la peau dans des compositions auto-bronzantes.

2. Utilisation selon la revendication 1, qui a trait à des composés de formule : 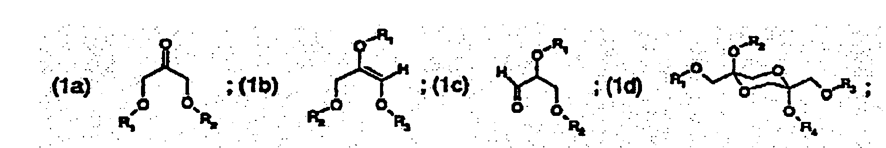 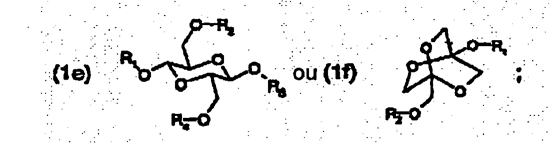 dans lesquelles
R₁, R₂, R₃ et R₄ représentent chacun, indépendamment les uns des autres, un atome d'hydrogène ou un radical de formule 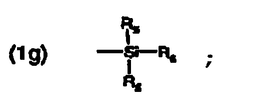
R₅ représente un groupe alkyle en C₁ à C₅ ; aryle ; aryle en C₁ à C₃ ; et
R₆ représente un groupe alkyle en C₁ à C₁₆ ;
où
dans les formules (1a) à (1f), au moins un des radicaux R₁, R₂, R₃ et R₄ représente un radical de formule (1g).

3. Utilisation selon la revendication 2, qui a trait à des composés de formule : 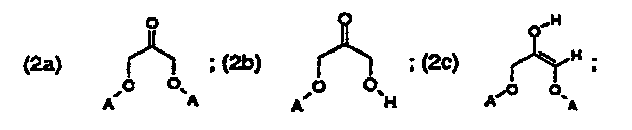 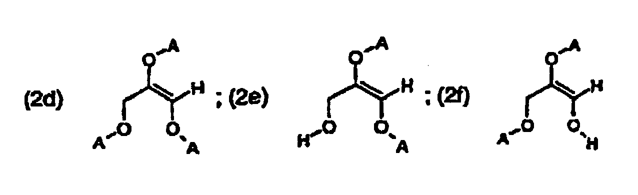 dans lesquelles
A représente un radical de formule (1g).

4. Utilisation selon la revendication 2, qui a trait à des composés de formule : 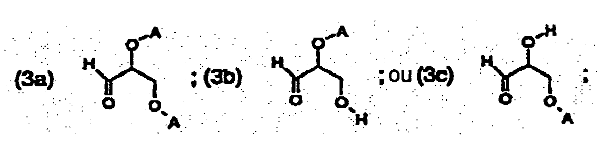 dans lesquelles
A représente un radical de formule (1g).

5. Utilisation selon la revendication 2, qui a trait à des composés de formule : 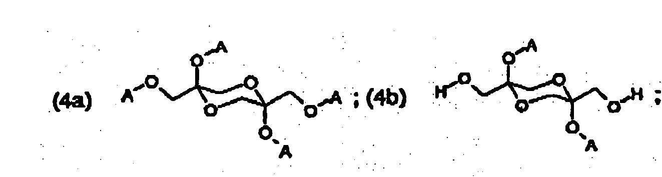 ou 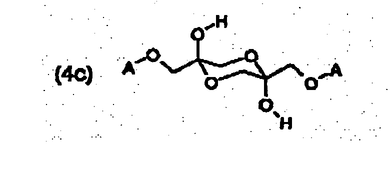 dans lesquelles
A représente un radical de formule (1g).

6. Utilisation selon la revendication 2, qui a trait à des composés de formule : 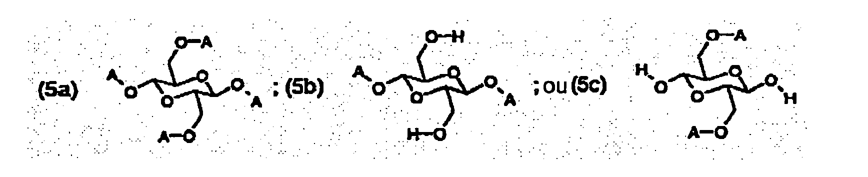 dans lesquelles
A représente un radical de formule (1g).

7. Utilisation selon la revendication 2, qui a trait à des composés de formule : 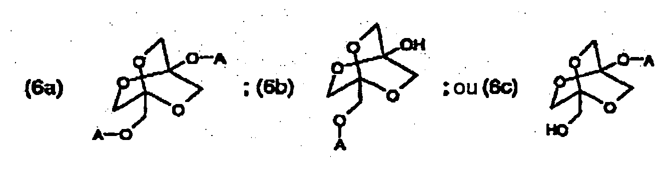 dans lesquelles
A représente un radical de formule (1g).

8. Utilisation selon l'une quelconque des revendications 2 à 7, dans laquelle, dans la formule (1g), R₅ représente un groupe méthyle ou éthyle.

9. Utilisation selon la revendication 1 ou 2, qui a trait au composé de formule

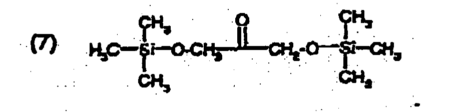

10. Utilisation selon la revendication 1 ou 2, qui a trait au composé de formule

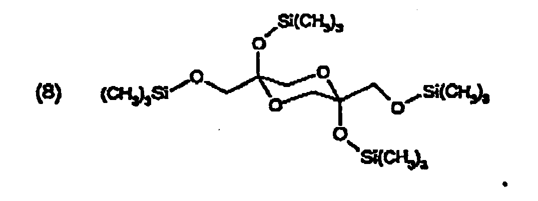

11. Préparation cosmétique comprenant
2% à 15% en poids d'un précurseur selon la revendication 1, et des véhicules ou adjuvants cosmétiques habituels.

12. Préparation cosmétique selon la revendication 11, qui est exempte d'eau.

13. Préparation cosmétique selon la revendication 11 ou 12, qui comprend en outre un filtre anti-UV.
